Europäisches Patentamt

European Patent Office

Office européen des brevets

(11) Publication number: **0 095 351**
**B1**

(12) **EUROPEAN PATENT SPECIFICATION**

(45) Date of publication of patent specification: **08.11.89**

(21) Application number: **83302916.8**

(22) Date of filing: **20.05.83**

(51) Int. Cl.⁴: **C 12 N 15/00,** C 07 K 7/36,
C 07 H 21/04, C 12 P 21/02,
C 12 N 1/00, C 12 N 5/00

(54) A precursor of a C-terminal amidated peptide and production thereof.

(30) Priority: **20.05.82 JP 86181/82**

(43) Date of publication of application:
**30.11.83 Bulletin 83/48**

(45) Publication of the grant of the patent:
**08.11.89 Bulletin 89/45**

(84) Designated Contracting States:
**AT BE CH DE FR GB IT LI LU NL SE**

(56) References cited:
EP-A-0 070 675

INT. CONGR. SER. EXCERPTA MED., vol. 540,
1981; J.JACOBS et al.: "Calcitonin precursors",
pp. 25-34

PROC. NATL. ACAD. SCI., vol. 77, no. 8, August
1980 (US); S.G.AMARA et al.: "Characterization
of rat calcitonin mRNA", pp. 4444-4448

CHEMICAL ABSTRACTS, vol. 95, no. 15, 21
October, 1981, p. 199, no. 127640a, Columbus,
OH (US); J.W.JACOBS et al.: "Calcitonin
messenger RNA encodes multiple
polypeptides in a single precursor"

(73) Proprietor: **SUNTORY KABUSHIKI KAISHA, also
known as SUNTORY LTD.**
**1-40, Dojimahama 2-chome**
**Kita-ku Osaka530 (JP)**

(72) Inventor: **Tanaka, Shoji**
**8-46, Minamikaneden-cho 1-chome**
**Suita Osaka 564 (JP)**
Inventor: **Ohsuye, Kazuhiro**
**18-7, Inaba-cho**
**Ibaraki Osaka 567 (JP)**
Inventor: **Kubota, Ichiro**
**15-14, Aoyamadai 4-chome**
**Suita Osaka 565 (JP)**
Inventor: **Ohnuma, Norio**
**19-401, Nara-cho**
**Ibaraki Osaka 567 (JP)**
Inventor: **Noguchi, Teruhisa**
**8-11, Kugenuma-kaigan 2-chome**
**Fujisawa Kanagawa 251 (JP)**

(74) Representative: **Burford, Anthony Frederick
et al**
**W.H. Beck, Greener & Co. 7 Stone Buildings**
**Lincoln's Inn**
**London WC2A 3SZ (GB)**

## EP 0 095 351 B1

**Description**

This invention relates to a gene for producing precursor of calcitonin, plasmids containing such genes and methods of producing a calcitonin.

Among C-terminal amidated peptides, some are pharmacologically active. For instance calcitonin is a peptide composed of 32 amino acid residues with the C-terminal being in the amide form and is used for the treatment of hypercalcemia, osteoporosis and Bahcet disease of the bone or for promoted osteogenesis. The amide form C-terminal structure is considered to be essential to activity development.

So far, pig, ox, sheep, human, rat salmon and eel calcitonins have been isolated and the structure of each calcitonin has been elucidated. Among them, human, eel, pig and salmon calcitonins are now available on the market for therapeutic use. These are either extraction products from animal bodies or chemically synthesized products. However, the calcitonin content in animal bodies is low and the synthesis is difficult.

It would be desirable to produce calcitonin on a large scale and at a low cost by using gene manipulation technology. However, it has been difficult to produce C-terminal amidated peptides.

It has been disclosed (Int. Cong. Ser. Excerpta Med. Vol. 540, 1981, pages 25 to 34; J. Jacobs et al: "Calcitonin precursors") that calcitonin is first synthesized as part of a substantially higher molecular weight precursor in which calcitonin is contained near the COOH-terminus flanked by glycine. It is disclosed that glycine is apparently needed for the enzymatic amidation of proline to prolinamide.

The paper discloses also the formation of a recombinant DNA by reverse transcription from isolated polyadenylated RNA and the cloning of the recombinant DNA in a plasmid.

WO 83/04028 describes chemically synthesized nucleotide fragments for human calcitonin in which the eighth amino acid residue is Met. It also describes a DNA sequence coding for a 32 amino acid calcitonin analog in which the eighth residue is Val.

"Nature", vol. 295, 28th January 1982, pp 345-347 discloses an amino acid sequence for human calcitonin.

The present invention provides a gene comprising a chemically synthesized polynucleotide which comprises the following sequence:

5'

TGT GGT AAC CTG AGC ACC TGT GTG CTG GGT ACC TAC ACC CAG

ACA CCA TTG GAC TCG TGG ACA CAC GAC CCA TGG ATG TGG GTC

GAT TTC AAC AAG TTC CAC ACC TTC CCG CAG ACC GCT ATC GGT

CTA AAG TTG TTC AAG GTG TGG AAG GGC GTC TGG CGA TAG CCA

3'

GTT GGT GCC CCG GGT AAG AAA CGC

CAA CCA CGG GGC CCA TTC TTT GCG,

the said sequence coding for the amino acid sequence represented by the formula:

Cys-Gly-Asn-Leu-Ser-Thr-Cys-Val-Leu-Gly-Thr-Tyr-

Thr-Gln-Asp-Phe-Asn-Lys-Phe-His-Thr-Phe-Pro-Gln-

Thr-Ala-Ile-Gly-Val-Gly-Ala-Pro-Gly-Lys-Lys-Arg.

The precursor resulting from addition of glycine (Gly) to the amino acid which is to be amidated and further addition of Lys-Lys-Arg produces pharmacological effects. The reason is presumably that the precursor is cleaved and modified *in vivo* under enzymatic actions to form the C-terminal adidated peptide.

2

Calcitonin can be produced using such a gene by the method to be described hereinafter.

It is preferable that a codon for methionine is added to the head of the nucleotide sequence of the gene and a translation-terminating codon to the tail thereof. Furthermore, it is preferable that a restriction enzyme-cleavage site is further added to each end; for instance, an *Eco*RI cleavage site upstream (on the 5' site) and a *Bam*HI cleavage site downstream (on the 3' side).

Furthermore, it is possible to introduce one or more restriction enzyme cleavage sites, for instance one or more *Rsa*I, *Kpn*I, *Sma*I, and/or *Xma*I site or sites, into the nucleotide sequence.

The invention includes a plasmid containing an *Escherichia coil* alkaline phosphatase or beta-galactosidase gene, which comprises a promoter region and a structural gene of the gene, one end of the gene having an EcoRI cleavage site and the other end having a BamHI cleavage site, and a gene comprising the following sequence:

5'

TGT GGT AAC CTG AGC ACC TGT GTG CTG GGT ACC TAC ACC CAG

ACA CCA TTG GAC TCG TGG ACA CAC GAC CCA TGG ATG TGG GTC

GAT TTC AAC AAG TTC CAC ACC TTC CCG CAG ACC GCT ATC GGT

CTA AAG TTG TTC AAG GTG TGG AAG GGC GTC TGG CGA TAG CCA

3'

GTT GGT GCC CCG GGT AAG AAA CGC

CAA CCA CGG GGC CCA TTC TTT GCG,

the said sequence coding for the following amino acid sequence;

Cys-Gly-Asn-Leu-Ser-Thr-Cys-Val-Leu-Gly-Thr-Tyr-

Thr-Gln-Asp-Phe-Asn-Lys-Phe-His-Thr-Phe-Pro-Gln-

Thr-Ala-Ile-Gly-Val-Gly-Ala-Pro-Gly-Lys-Lys-Arg.

The invention includes E. Coli transformed with such a plasmid.

The invention further includes a method of producing a precursor of calcitonin, which comprises (a) cultivating *Escherichia coli* cells transformed with a plasmid comprising an *Escherichia coli* alakaline phosphatase or beta-galactosidase structural gene attached to a synthetic gene comprising, in sequence, a codon for methionine, a polynucleotide represented by the sequence of

5'

TGT GGT AAC CTG AGC ACC TGT GTG CTG GGT ACC TAC ACC CAG

ACA CCA TTG GAC TCG TGG ACA CAC GAC CCA TGG ATG TGG GTC

GAT TTC AAC AAG TTC CAC ACC TTC CCG CAG ACC GCT ATC GGT

CTA AAG TTG TTC AAG GTG TGG AAG GGC GTC TGG CGA TAG CCA

3'

GTT GGT GCC CCG GGT AAG AAA CGC

CAA CCA CGG GGC CCA TTC TTT GCG

3

Referring to the drawings, the figures are concerned with an embodiment of the present invention, which is to be described later by way of example.

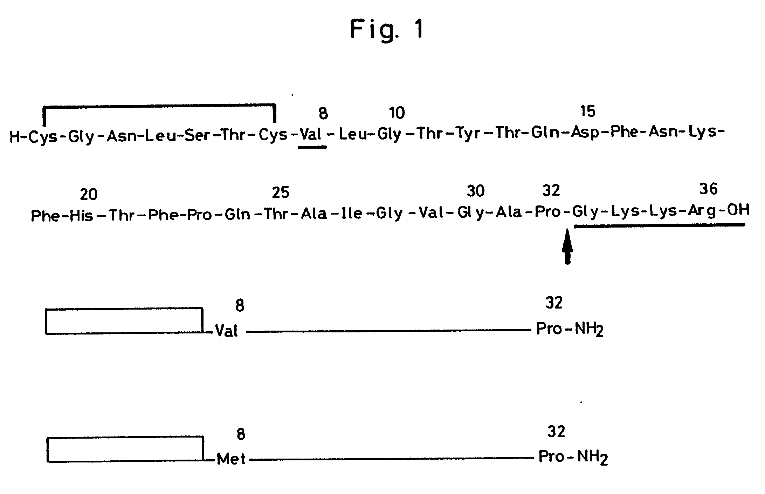

Thus, Figure 1 illustrates, in the upper row, the amino acid sequence on which the synthetic gene designing is based and, in the lower row, the structure of mature calcitonin, with the portions in the lower row which are the same as those in the upper row being represented by line segments,

Figure 2 shows the design of a gene, HPCT,

Figure 3 shows the chemically synthesized fragments (F1 to F20) of the HPCT (1—36) gene,

Figure 4 shows the process of immobilising a 3′-terminal mononucleotide unit on an aminomethylated polystyrene resin,

Figure 5 shows the structure of 3′-phosphodiester dimer (or trimer) blocks,

Figure 6 shows the process for constructing a recombinant plasmid containing the HPCT gene,

Figure 7 shows the HPLC elution pattern for HPCT produced by a bacterial strain transformed with the above-mentioned plasmid,

Figure 8 shows the HPLC elution pattern for the fraction presenting a peak in Figure 7, and

Figure 9 shows the amino acid sequence of HPCT isolated by the above HPLC.

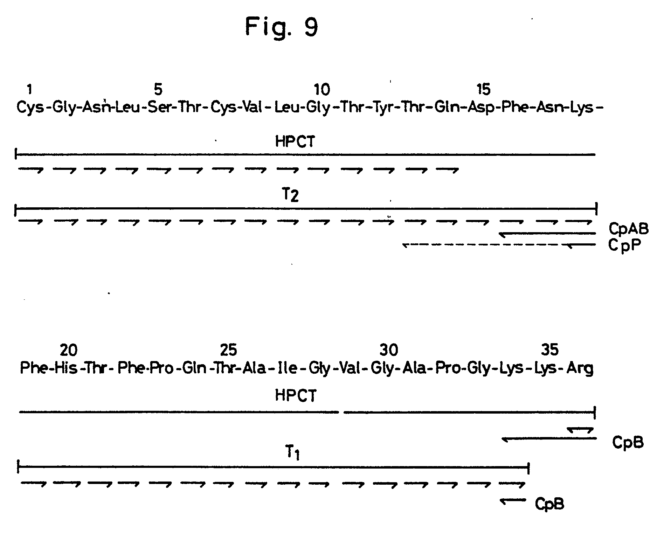

In Figure 9, Cp means carboxypeptidase, $\rightarrow$ denotes the amino acid sequence as determined by the Edman's degradation method $\leftarrow$ denotes the carboxy terminal portion amino acid sequence as determined by the carboxypeptidase method, $\leftarrow$-- denotes the carboxy terminal portion amino acid sequence as estimated by the carboxypeptidase method, and $\leftarrow\tau$ denotes the carboxy as determined by the hydrazine decomposition method.

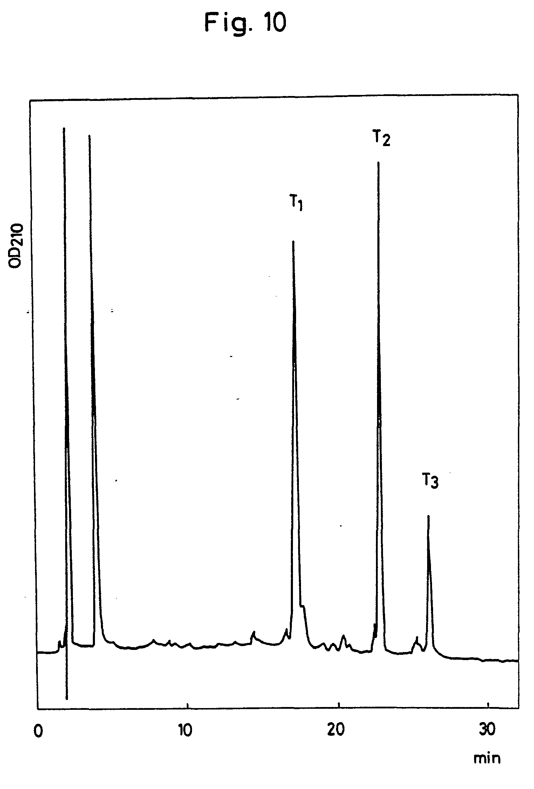

Figure 10 shows the HPLC elution pattern for the trypsin digestion product from HPCT, and

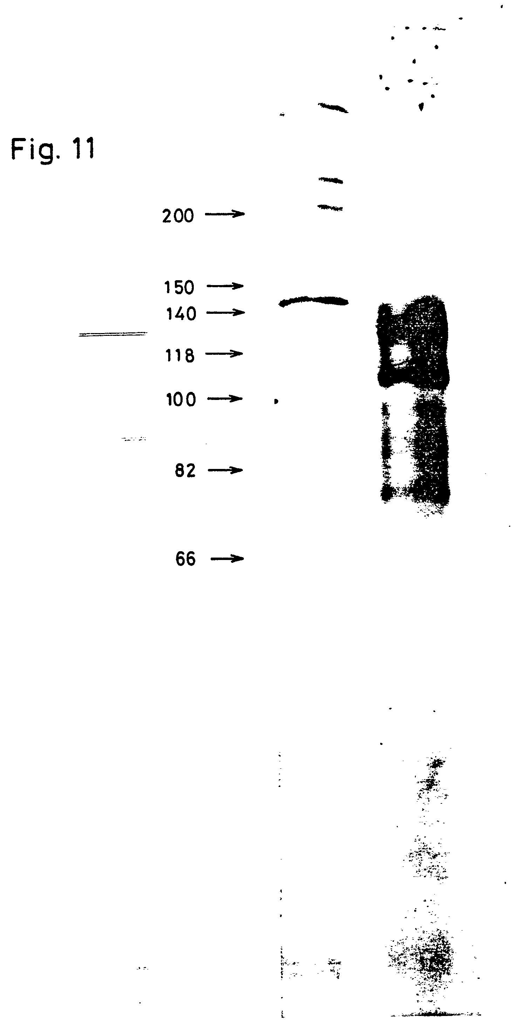

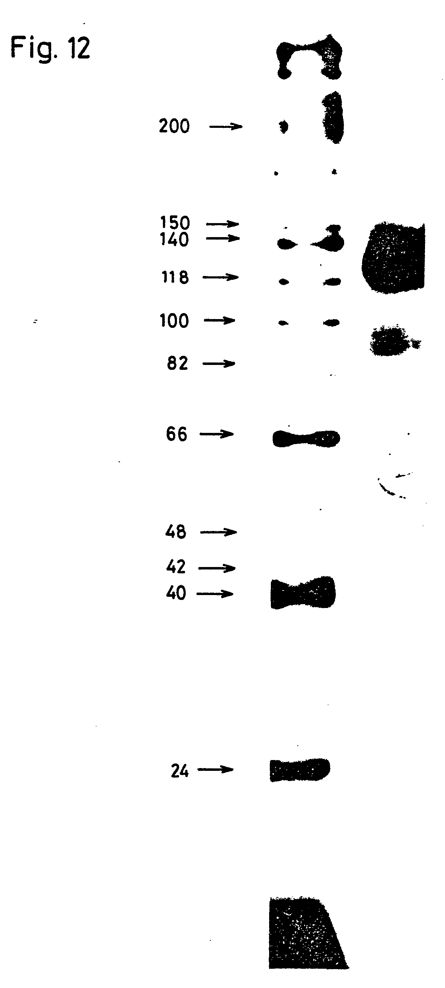

Figures 11 and 12 are the 8% polyacrylamide gel electrophoresis mappings for the gene synthesized from the oligonucleotide fragments F1 to F20.

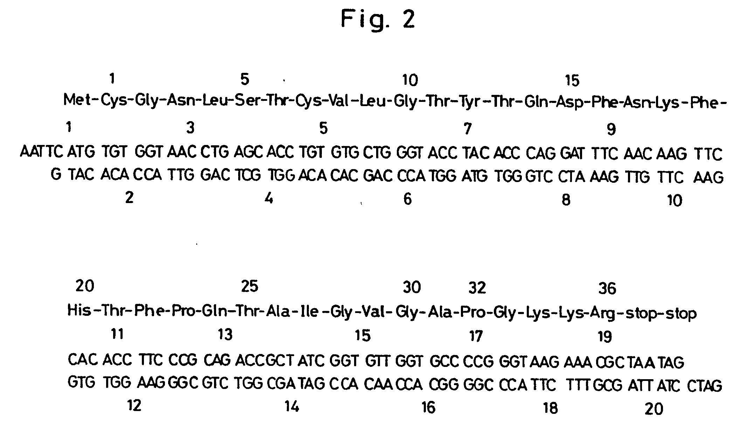

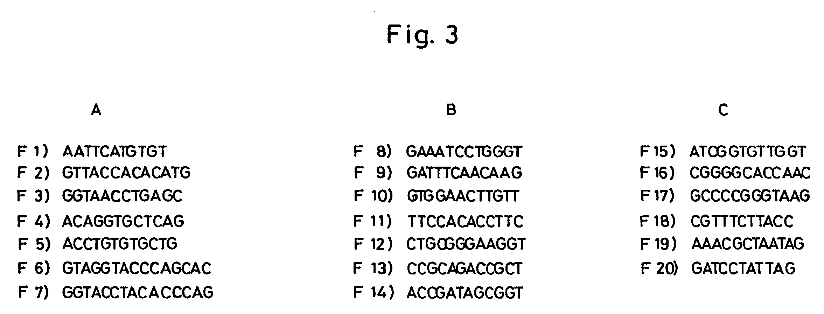

A preferred example of the design of the double-stranded gene DNA is given in Figure 2. The chemical synthesis of said gene is performed by first synthesizing the twenty DNA fragments F1 to F20 shown in Figure 3 by the solid phase method (Miyoshi, K., Nucleic Acids Res., 8, 5507, 1980) and then preparing the double-stranded DNA from these fragments by the ligase reaction method.

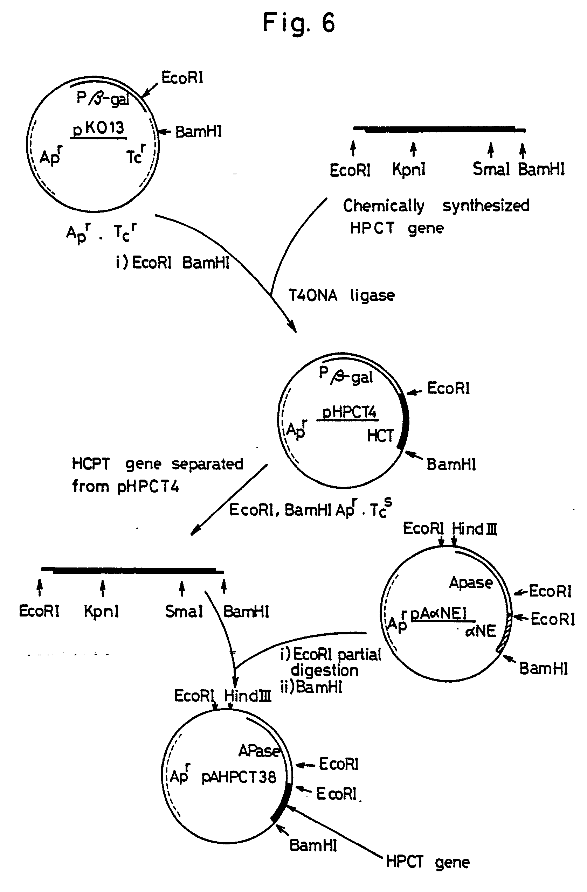

Plasmids may be produced by connecting the above gene to a structural gene (e.g. APase or beta-galactosidase) in an operon containing a promoter-region derived from a unicellular organism, such as a microbe (e.g. bacterium, yeast, mold) or an animal cell such as monkey kidney cell, in a manner such that correct reading frame placement can be obtained. Examples are pAHPCT38 as illustrated in Figure 6 and pHPCT4 as shown in Figure 6. pHPCT4 can be obtained by inserting the above chemically synthesized gene in between the *Eco*RI and *Bam*HI cleavage sites of pK013 (cf. Japanese Patent Application Sho 56—163303), while pAHPCT38 can be obtained by inserting the above chemically synthesized gene in between the *Eco*RI and *Bam*HI cleavage sites of the plasmid pA NE1 (cf. Japanese Patent Application Sho 56—17043) derived from an *Escherichia coli* alkaline phosphatase (APase) gene-containing plasmid pBR322.

A transformant strain WA802/pHPCT may be obtained by transformation of an *Escherichia coli* strain K12 WA802 with the plasmid pHPCT4, and a transformant strain E15/pAHPCT may be obtained by transformation of an *Escherichia coli* strain K12.E15 with the plasmid pAHPCT38.

The strain E15/pAHPCT38 has been given a code name SBMC138 and deposited with the Fermentation Research Institute, Agency of Industrial Science and Technology under Deposit N.FERM BP 283.

A precursor of calcitonin may be prepared by cultivating host cells transformed with a plasmid of the invention and thereby causing formation of the above peptide as a protein component in the culture broth.

As example of the transformed host cells is the above-mentioned E15/pAHPCT. Taking this as an example, the method of cultivation is described. First, in a typical case, said transformant is cultured in a high phosphoric acid content medium and then transferred to a low phosphoric acid content medium, whereby APase is induced and the desired peptide precursor is formed as a protein component is the cells.

Preferred procedure and conditions of cultivation should preferably be selected depending on the plasmid species and host cell species.

After cultivation, the cells are separated from the culture, and treated with formic acid-cyanogen bromide. The desired fraction containing the desired peptide can be collected, for example, by density gradient fractional elution on an SP-Sephadex column using a pyridine-acetic acid buffer and purified by HPLC.

The thus-obtained precursor of C-terminal amidated peptide is as active as the C-terminal amidated peptide. For instance, the precursor HPTC shown in the upper part of Fig. 1 showed calcitonin activity, markedly reducing the calcium level in the rat blood. The abbreviation HPCT stands for a calcitonin precursor in which Met[8] of human calcitonin is replaced by Val.

## Example

Chemical synthesis of HPCT gene fragments

The chemical synthesis of 20 oligodeoxyribonucleotides respectively designated $F_1$ through $F_{20}$ in Fig. 3, except for the improvement mentioned hereinafter, was carried out basically by the solid phase method reported by Ken-ichi Miyoshi et al [Nucl. Acids Res. 8, 5507, (1980)].

The outline of chemical synthesis of these oligodeoxyribonucleotides by the improved technology is as follows.

4

EP 0 095 351 B1

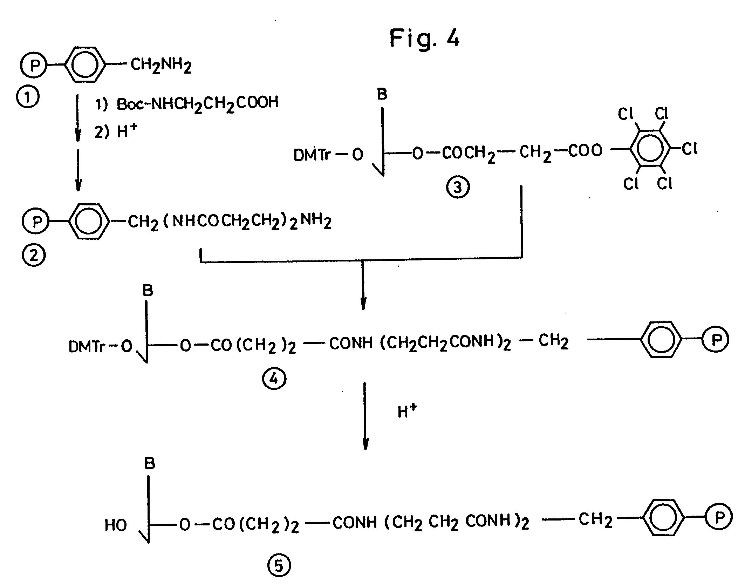

a) First, as shown in Fig. 4, the 3'-end mononucleoside unit was bound to an aminomethylated polystyrene resin. Thus, the aminomethylated polystyrene resin (1) shown in Fig. 4 is a resin cross-linked with 1% divinylbenzene, and the resin (2) was prepared by adding 2 units of beta-alanine to (1).

Then, three different compounds (3) (B represents thymine, N-benzoylcytosine or N-isobutyrylguanine) were reacted with the above resin (2) to provide 3 different polystyrene resins (4) each carrying the 3'-end nucleoside.

The following is an example of synthesis of the same resin (4) relevant to thymine for B in Fig. 4.

Fifteen grams of aminomethylated polystyrene·HCl (divinylbenzene 1%, 100—200 mesh, NR$_2$: 0.63 m mol/g was swollen well with 75 ml of CH$_2$Cl$_2$ and N-t-BOC-beta-alanine (1.01 g, 11.34 m moles) was added. The condensation reaction was conducted in the presence of DCC (2.34 g, 11.34 m moles) at room temperature for 3 hours. The resin was filtered through a glass filter and washed 4 times with 150 ml portions of CH$_2$Cl$_2$, 4 times with 150 ml portions of DMF and finally 4 times with 50 ml portions of pyridine. Then, the resin was treated with 75 ml of 25% acetic anhydride-pyridine for 30 minutes to acetylate the unreacted amino groups. Thereafter, the resin was treated with 90 ml of 20% CF$_3$COOH—CH$_2$Cl$_2$ at room temperature for 25 minutes to eliminate the t-BOC groups. The resin was washed with 150 ml of 5% triethylamine—CH$_2$Cl$_2$ and further washed 4 times with 50 ml portions of CH$_2$Cl$_2$. The above procedure was repeated for a second time to give the resin (2) of Fig. 4, i.e. the amino-methylated polystyrene resin having two β-alanine units. In 30 ml of DMF was suspended by 5 g of the above resin [Fig. 4, (2)], followed by the addition of 5 m mole of compound (3) [Fig. 4, (3)] (where B means N-thymine) and 500 mg of triethylamine. The mixture was shaken at room temperature for 12 hours. After the reaction, the resin was filtered through a glass filter, washed with DMF and, then, with pyridine, and finally treated with 75 ml of 10% phenyl isocyanate-pyridine for 3 hours to protect the unreacted amino groups. The resin was subsequently washed with pyridine-methanol and dried under reduced pressure in the presence of P$_2$O$_5$, whereby the resin (4) of Fig. 4 (B means thymine) was obtained.

Using a portion of this resin (Fig. 4, (4), where B is thymine), the amount of 3'-end nucleoside secured to 1 gram of the resin was determined by the assay method of M. J. Gait et al [Nucl. Acids Res. 8, 1081 (1980)]. The result was 0.153 m mole. The resin (4) wherein B is N-benzoylcytosine or N-isobutylquanine was also prepared in the same manner as above. The amount of 3'-end nucleoside secured to 1 g of resin (4) was 0.150 m mole for B = N-benzoylcytosine and 0.147 m mole for B = N-isobutylguanine.

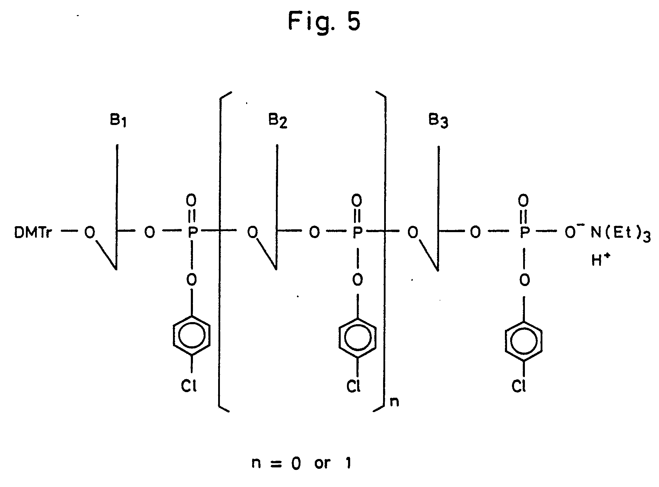

b) Using these three kinds of resin (4) and the 3'-phosphodiester dimer block (Fig. 5, n = 0) and 3'-phosphodiester trimer block (Fig. 5, n = 1), 20 oligodeoxyribonucleotides F$_1$ through F$_{20}$ having the specified predetermined base sequences. Thus, the resin (4) was treated with 2% benzenesulfonic acid (CHCl$_3$-MeOH = 7:3, v,v) to remove the dimethoxytrytyl group (briefly, DMTr) and, then, 4 equivalents of 3'-phosphodiester dimer (Fig. 5, n = 0) or 3 equivalents of 3'-phosphodiester trimer (Fig. 5, n = 1) per equivalent of the 3'-nucleoside unit secured to resin (5) was sequentially condensed with the aid of 20 to 50 equivalents of mesitylenesulfonyltetrazolide (MsTe). The excess 3'-phosphodiester block and condensing agent could be easily removed by filtering the reaction mixture through a glass filter.

Then, to protect the unreacted 5'-hydroxy group, the resin was treated with 10% acetic anhydride-pyridine at room temperature for 1 hour, whereby the unreacted 5'-hydroxy groups were acetylated. The resin was then treated with 2% benzenesulfonic acid (CHCl$_3$-MeOH = 7:3, v/v) to remove the 5'-end DMTr group and, then, the next condensation reaction was conducted. The above procedure was repeated until the required length was obtained.

An exemplary sequence of synthesis is given below with reference to the F$_3$ fragment.

200 mg of resin (4) [Fig. 4, (4), B = N-benzoylcytosine] was treated twice with 20 ml of 2% benzenesulfonic acid (CHCl$_3$-MeOH = 7:3, v/v) (hereinafter abbreviated to 2% BSA) at room temperature for 1 minute each to give the resin (5) free from DMTr. To this resin (5) was added 90 µ moles of the 3'-phosphodiester trimer block GAG (Figs. 5, n = 1, B$_1$ = N-isobutyrylguanine, B$_2$ = N-benzoyladenine, B$_3$ = N-isobutyrylguanine) and the azotropic distillation with 3 ml of pyridine was repeated 3 times. Then, 2 ml of pyridine was added to the resin and the condensation reaction using 1 m mole of the condensing agent MsTe was carried out at room temperature for 2 hours. After the reaction, the excess 3'-phosphodiester trimer and condensing agent could be easily removed by filtering the reaction mixture through a glass filter and washing the resin well with pyridine. Then, to protect the unreacted 5'-hydroxy groups, the resin was suspended in 20 ml of 10% acetic anhydride-pyridine and the suspension was shaken at room temperature for 1 hour. After this reaction, the resin was separated by filtration through a glass filter and washed well with pyridine and CHCl$_3$ in the order mentioned. Then, the resin was treated twice with 20 ml of 2% BSA (CHCl$_3$-MeOH = 7:3, v/v) for 1 minute each, whereby the DMTr group was removed from the resin. The resin was washed well with CHCl$_3$ and pyridine in that order. The above procedure was repeated until the required length was obtained. Thus, to synthesize the oligodeoxyribonucleotide of F$_3$, the next 3'-phosphodiester blocks CCT, AA and GGT were sequentially employed. By these sequential condensation reactions, a completely protected oligodeoxynucleotide-resin having the base sequence of F$_3$ was obtained. The other fragments F$_1$, F$_2$ and F$_4$ to F$_{20}$ were also produced in the same manner except that different combinations of nucleotides were employed.

c) The severence of the completely protected oligodeoxyribonucleotide having such a definite base sequence from the resin and the complete deprotection thereof were carried out by treating the resin with

5

concentrated aqueous ammonia-pyridine (2:1, v/v) at 55°C for 10 hours and, then, with 80% acetic acid at room temperature for 10 minutes.

This deprotection procedure is described below with reference to $F_3$ fragment.

In a sealed tube, 50 mg of the $F_3$-resin was shaken with 1 ml of pyridine and 2 ml of concentrated aqueous ammonia at 55°C for 10 hours, at the end of which time the resin was filtered off and the filtrate was concentrated under reduced pressure. To the residue was added toluene and the azeotropic distillation was repeated to remove the pyridine.

The residue was treated with 2 ml of 80% acetic acid at room temperature for 10 minutes, whereby a completely deprotected oligodeoxyribonucleotide was obtained.

d) The isolation and purification of the final product was carried out by high performance liquid chromatography (briefly, HPLC). Thus, ALTEX Model 110 A Liquid Chromatograph, a linear gradient method using Solvent A (0.05 M $KH_2PO_4$, pH 4.0) and a Solvent B (0.05 M $KH_2PO_4$-1.0 M KCl, pH 4.0), and a Permaphase AAX (Du Pont) column (0.21 × 50 cm) were employed. The gradient was established by adding 3% of Solvent B to Solvent A at the intervals of 1 minute. Elution was carried out at 58°C and a flow rate of 2 ml/min. The fractions containing the desired compound were pooled, desalted by dialysis and lyophilized. Each of the fragments thus obtained was further purified by HPLC on a linear gradient of Solvent A (0.01 M EDA/2.5% acetonitrile, pH 7.0) and Solvent B (0.01 M EDA/25% acetonitrile, pH 7.0) in a μ-Bondapak $C_{18}$ column (0.4 × 25 cm). The gradient was established by adding 3% of Solvent B to Solvent A at intervals of 1 minute. Elution was carried out at room temperature and a flow rate of 2 ml/min. The fractions containing the desired compounds were respectively pooled and lyophilized to obtain 20 DNA fragments $F_1$ through $F_{20}$.

e) The homogeniety of the 20 completely deprotected oligodeoxyribonucleotides $F_1$ through $F_{20}$ thus obtained was confirmed by labeling their 5'-ends with [gamma-$^{32}$P]ATP using $T_4$ polynucleotide kinase and carrying out polyacrylamide gel (20%) electrophoresis. The base sequence of each oligodeoxyribonucleotide was confirmed by the two-dimensional mapping method [Bambara, J.E. et al (1974) Nucl. Acids, Res. 1, 331]. Thus, the 5'end was labeled with [gamma-$^{32}$P]ATP using $T_4$ polynucleotide kinase and, then, partial hydrolysis was carried with nuclease. This partial hydrolysate was subjected to cellulose acetate electrophoresis (pH 3.5) and DEAE-cellulose homochromatography.

Ligation of HPCT gene fragment

The 5'-ends of the chemically synthesized 18 oligonucleotide fragments ($F_2$ through $F_{19}$; Fig. 3) were phosphorylated and after $F_1$ and $F_{20}$ fragments were annealed, they were ligated by means of T4 DNA ligase to synthesize a gene corresponding to HPCT consisting of 122 base pairs. Details of the procedure are given below.

Excepting $F_1$ and $F_{20}$, 4.2 μg of each of the remaining 18 fragments was dissolved in 22 μl of distilled water and the solution was heated at 90°C for 2 minutes and, then, immediately cooled to 0°C. To this aqueous solution was added 10 μCi of γ[$^{32}$P] ATP (5100 Ci/m mole). It was then adjusted to give a 50 mM Tris-HCl (pH 7.5)-10 mM $MgCl_2$·2 mM spermine-100 mM KCl-10 mM DTT oligonucleotide kinase buffer solution. Then, 3 units of polynucleotide kinase was added to make a total of 30 μl. By conducting the reaction at 37°C for 15 minutes, the 5'-ends were labeled with $^{32}$P. Then, to phosphorylate all the 5'-ends, 4 n moles of ATP and 3 units of polynucleotide kinase were further added and the reaction was continued at 37°C for 45 minutes. The reaction was terminated by heating the mixture at 90°C for 5 minutes. 2.1 μg each of the above 18 phosphorylated fragments $F_2$ through $F_{19}$ and unphosphorylated fragments $F_1$ and $F_{20}$ were respectively mixed and dialyzed in a dialysis tube against distilled water at 4°C for 16 hours to remove the unreacted ATP and kinase buffer components. This DNA dialyzate was concentrated to dryness and dissolved in 14.5 μl of ligase buffer (20 mM tris-HCl, pH 7.6, 10 mM, $MgCl_2$). This solution was put in a 0.5 ml Eppendorf tube and heated at 95°C for 2 minutes. Thereafter the temperature was gradually lowered (to room temperature in 30 minutes) to anneal the 20 fragments. The reaction mixture was then held at 0°C. This DNA solution was made into a ligase buffer containing 0.4 mM of ATP and 10 mM of DTT. Then, 30 units of T4 DNA ligase was added to make a total volume of 80 μl and the reaction was allowed to proceed at 11°C for 16 hours. A portion of the reaction mixture was taken and subjected to electrophoresis with 18% polyacrylamide gel. Using two kinds of gels, one containing 7 M of urea and the other not containing urea, electrophoresis was carried out and the reaction product was analyzed by radioautography. It was found that a DNA corresponding to 122 base pairs had been produced at the rate of about 20 percent (Figs. 11 and 12). Then, the entire amount of the above reaction mixture was fractionated by electrophoresis through 8% polyacrylamide gels. After the 122 base pairs of DNA were confirmed by radioautography, the corresponding portion of the gel was cut out and placed in dialysis tubes. Each tube was filled with 9 mM Tris-borate (pH 8.3)-0.25 mM EDTA buffer and after closing both ends of the tube, electrophoresis was conducted in the buffer at a constant voltage of 200-V for 3 hours to elute the DNA from the gel. The DNA solution was taken out from the dialysis tube and lyophilized.

The lyophilisate was dissolved in 100 μl of 0.3 M sodium acetate (pH 7.0), and 2.5 volumes of ethanol was added. The mixture was allowed to stand at −80°C for 30 minutes, at the end of which time it was centrifuged to recover the DNA sediments. The DNA thus obtained was dissolved in 30 μl of kinase buffer, 6 units of polynucleotide kinase and 0.8 nmol ATP were added, and the reaction was conducted at 37°C for an hour. Thereafter, 3 µl of 3 M sodium acetate was added, followed by 2.5 volumes of ethanol. There was thus obtained about 2 µg of the HPCT gene comprising 122 base pairs as the DNA precipitate.

Construction of recombinant plasmids

Fig. 6 illustrates the method of constructing recombinant plasmids containing the HPCT. The method is described hereinbelow in more detail.

(A) Construction of plasmid pHPCT

The method of construction of the plasmid pK013 is as distinctly disclosed in the specification of Japanese Patent Application No. 163303/1981. The plasmid pK013 is a plasmid having most of the beta-galactosidase genes from the promoter region of the *Escherichia coli* lactose operon. The plasmid has one EcoRI and one BamHI cleavage site, and therefore genes obtained chemically or from natural sources and having EcoRI and BamHI cohesive ends can be easily inserted into the plasmid pK013. Furthermore, it is possible to cause the genes inserted downstream to the EcoRI cleavage site to express in terms of hybrid proteins with beta-galactosidase under the control of the lactose promoter. Following decomposition of the hybrid proteins with cyanogen bromide the desired peptides can be separated and purified.

Thus, 5 µg of pK013 was reacted at 37°C for 60 minutes with 1 unit each of the restriction enzymes EcoRI and one BamHI in 40 µl of 1 × TA mixture (containing 33 mM Tris-acetate buffer, pH 7.6 66 mM potassium acetate, 10 mM magnesium acetate and 0.5 mM dithiothreitol). The reaction mixture was heated at 65°C for 30 minutes so as to inactivate the enzymes. Then, 0.7% agarose electrophoresis was carried out and large DNA fragments were recovered by electrophoresis. The resulting EcoRI/BamHI-cleaved fragment DNA of pK013 (1 µg) and 0.6 µg of the 5'-OH phosphorylated HPCT gene previously obtained were dissolved in 40 µl of T4 DNA ligation mixture (20 mM Tris-HCl buffer pH 7.6, 10 mM MgCl$_2$, 10 mM DTT, 0.4 mM ATP) and reacted with 2 units of T4 DNA ligase at 5°C for 16 hours. To this reaction mixture was added 2.5 volumes of cold ethanol to give a DNA precipitate. This DNA was dissolved in 15 µl of distilled water and using 10 µl of the solution, it was introduced into *E. coli* WA 802. The transformant was cultivated on a nutrient agar medium containing 40 µg/ml of ampicillin at 37°C overnight and the colony formed was replicated on a nutrient agar medium containing 10 µg/ml of tetracycline to obtain tetracycline-sensitive colonies. The ampicillin-resistant, tetracycline-sensitive transformants thus obtained were designated as WA802/pHPCT1 through WA802/pHPCT5. The DNA base sequence of the small EcoRI/BamHI cleaved DNA fragment obtained from WA802/pHPCT4 was analyzed by the procedure described below to find that the pHPCT4 plasmid had the DNA base sequence of the desired HPCT gene.

In this WA802/pHPCT4 strain, HPCT is expressed as a β-galactosidase-HPCT hybrid protein under the control of the lactose promotor and the HPCT can be separated as a complete peptide desired from this hybrid protein by methionine decomposition with cyanogen bromide. However, since the β-galactosidase protein contains 23 methionine residues, a number of unnecessary impurity peptides are produced in the cyanogen bromide decomposition, thus interfering with the separation and purification of the desired peptide. Therefore, we developed as a vector free from the above disadvantages a plasmid derived from the *E. coli* alkaline phosphatase gene and established its usefulness (Japanese Patent Application No. 170543/1981). Therefore, in the thought that for the separation and purification of the HPCT peptide from *Escherichia coli,* it is more desirable to construct a plasmid by recombination of the alkaline phosphatase gene-based plasmid with the HPCT gene, we performed the construction of such a plasmid as follows.

The HPCT base sequence in the plasmid carried by the above transformant strains was analyzed in the following manner.

HPCT base sequence

Each (150 µg) of the plasmid DNAs, pHPCT1, pHPCT2, pHPCT3 and pHPCT4, was digested with 200 units of the restriction enzyme EcoRI and 200 units of the restriction enzyme Sa/I, and a DNA fragment comprising about 400 base pairs inclusive of the PHCT gene was separated and purified. Then, the 5'-end was dephosphorylated with bacterial alkaline phosphatase, and the 5'-end was labeled with $^{32}$P using γ-[$^{32}$P]ATP and polynucleokinase. Thereafter, digestion was conducted with 100 units of the restriction enzyme BamHI, and a DNA fragment comprising 122 base pairs was separated and purified and subjected to base sequencing by the Maxam-Gilbert method (Proc. Natl. Acas. Sci. U.S.A., *74,* 560—564, 1977). As a result, it was confirmed that all the plasmids except for pHPCT1, had the base sequence as initially designed. In pHPCT1, the third member (C) of the codon (AAC) corresponding to the 17th amino acid of HPCT, namely asparagine, had been substituted by T. Nevertheless, the codon AAT also corresponds to the asparagine.

B) Construction of plasmid pAHPCT38

The method of constructing plasmid pAαNE1 is detailed in the specification of Japanese Patent Application No. 170543/1981. pAαNE1 is a plasmid prepared by incorporating an α-neoendorphin gene downstream of the EcoRI cleavage site of an *E. coli* alkaline phosphatase structural gene, and is a vector which expresses an alkaline phosphatase alpha-neoendorphin hybrid protein under the expression and modulation control of alkaline phosphatase. The procedure for construction of plasmid pAHPCT38 containing an HPCT gene in place of the alphaneoendorphin gene is described in detail below.

In 100 µl of 1 × TA mixture, 10 µl of plasmid pAαNE1 was partially cleaved using 10 units of the

restriction enzyme *Eco*RI at 37°C for 30 minutes. Then, 0.7% agarose gel electrophoresis was carried out and the DNA fragment cleaved by *Eco*RI at one site only was recovered by electrophoresis. The DNA fragment thus recovered was completely cleaved in 30 µl of 1 × TA using 5 units of the restriction enzyme *Bam*HI at 37°C for 60 minutes, after which 2.5 volumes of cold ethanol was added thereto so as to precipitate the DNA. This DNA precipitate was dissolved in 30 µl of distilled water. In 30 µl of T4 DNA ligation medium was dissolved 0.5 µg of this *Eco*RI/*Bam*HI-cleaved DNA fragment together with 0.5 µg of an HPCT gene DNA prepared from pHPCT4 using *Eco*RI and *Bam*HI, and ligation was conducted using 2 units of T4 DNA ligase at 5°C for 16 hours. To the reaction mixture was added 2.5 volumes of cold ethanol to give a DNA precipitate. This DNA precipitate was dissolved by addition of 10 µl of distilled water and introduced into *Escherichia coli* E15 [Hayashi et al: J. Biol. Chem. *239*, 3091, (1964)]. Each transformant strain was incubated on a nutrient agar medium containing 40 µg/ml of ampicillin at 37°C overnight. The 72 ampicillin-resistant strains obtained in the above manner were examined for the existence of *Sma*I-cleaved sites in plasmids. It was found that 3 strains are found to carry plasmids having *Sma*I-cleaved sites within the HPCT gene.

One of these strains was represented as E15/pAHPCT38 and used in the next separation and purification of HPCT peptide.

The above *Escherichia coli* E15/pAHPCT38 has been deposited with the Fermentation Research Institute, the Agency of Industrial Science and Technology under the deposit number of FERM BP 283.

Purification of a human calcitonin precursor from E. coli E15/pAHPCT38

In *E. coli* E15/pAHPCT38, the HPCT gene was inserted into the alkaline phosphatase structural gene. Therefore, HPCT was expected to be expressed as a protein hybridized with alkaline phosphatase. However, as shown in the initial HPCT gene design, by inserting methionine at the N-terminal of HPCT and by cleaving this alkaline phosphatase-HPCT hybrid protein with cyanogen bromide, HPCT can be easily obtained. Therefore, the separation and purification of HPCT from *E. coli* K12E15/pAHPCT38 was performed by the procedure described in detail hereinafter. As to the cultivation of *E. coli* K12E15/pAHPCT38 and the induction of the alkaline phosphatase-HPCT hybrid protein, these were conducted in accordance with the methods described in the specification of Japanese Patent Application No. 170543/1981.

The *E. coli* E15/pAHPCT38 strain was incubated in 100 ml of TG+20 (high phosphate medium) containing 40 µg/µl of ampicillin at 37°C for 16 hours and the culture was used to inoculate 10 l of TG+1 (low phosphate medium) and further incubated at 37°C for 24 hours. The cells were collected by centrifugation to recover 2.36 g of wet cells. The cells were suspended in 20 ml of a 70% formic acid solution containing 500 mg of cyanogen bromide and allowed to stand in the dark at room temperature for 24 hours.

Purification of HPCT from E. coli E15/pAHPCT1

The cyanogen bromide-treated cell suspension was lyophilized and after addition of 60 ml of distilled water, it was ultrasonicated at room temperature for about an hour. The resulting suspension was centrifuged (10,000 r.p.m., 15 min.) and with the supernatant kept undiscarded, the sediment was diluted with 30 ml of distilled water, ultrasonicated again and centrifuged. The supernatant was combined with the previous supernatant to give 90 ml. This solution was made up to 100 ml with 10 ml of distilled water and passed through an SP-Sephadex C-25 column (1.4 × 20 cm). The column was previously equilibrated with 0.025 M acetic acid and after application of the sample, it was washed with 230 ml of the same acetic acid solution and, then, with 200 ml of 1.5% pyridine-acetate buffer (briefly, PA buffer) (pH 4.47) and 160 ml of 2.5% PA buffer (pH 4.65). Thereafter, elution with 60 ml of 2.5% PA buffer (pH 4.65) and 60 ml of 3.75% PA buffer (pH 4.76) was carried out by the linear gradient method, followed by further elution with 60 ml of the latter buffer and still further elution on a linear gradient using 160 ml of 3.75% PA buffer (pH 4.76) and 160 ml of 5.0% PA buffer (pH 4.87), the eluate being collected in 6 ml fractions. At the interval of several tubes, a 100 µl portion of each fraction was subjected to HPLC analysis. (The conditions of HPLC are mentioned hereinafter.) The fractions corresponding to the main peaks on HPLC were respectively evaporated to dryness, dissolved in 150 ml of 6 N hydrochloric acid containing 0.1% phenol, and hydrolyzed at 110°C for 24 hours. The hydrolysate was evaporated to dryness and submitted to amino acid analysis (Hitachi #835—50 Amino Acid Analyzer) to monitor the state of elution from the column. As a result, the presence of HPCT was confirmed in the entire 2.5%—3.75% gradient eluate (total, 120 ml), the entire 3.75% PA buffer eluate (60 ml), and the first 10 fractions (60 ml) in the 3.75%—5.0% gradient eluate.

These HPCT-containing fractions were pooled and lyophilized, and the purification thereof by HPLC was carried out.

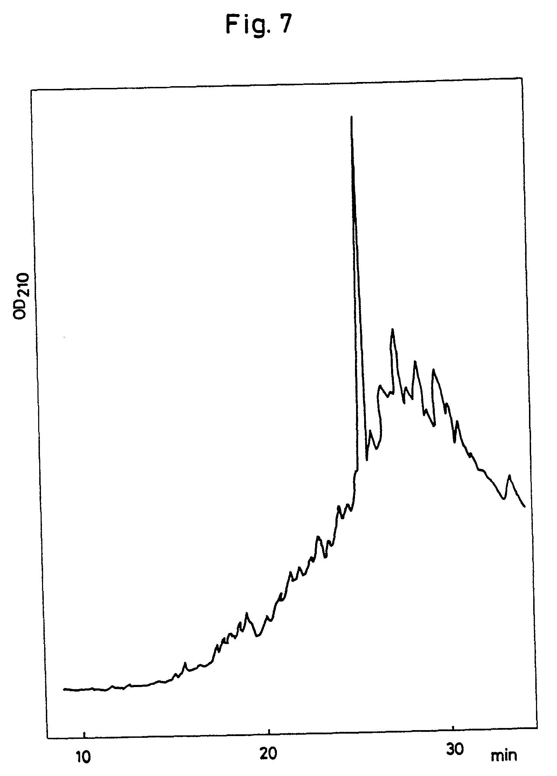

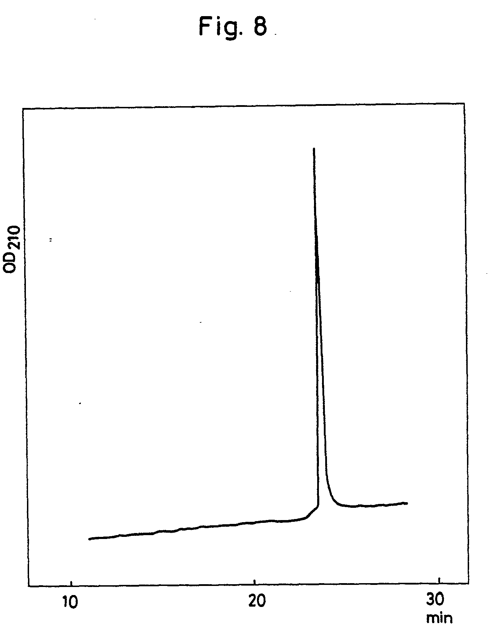

The above dried product was dissolved in 8.0 ml of 0.1 N acetic acid and 100 to 300 µl portions thereof were purified by means of µ-Bondapack $C_{18}$ (Waters) columns (0.39 × 30 cm). Elution was carried out by the linear gradient method using 0.1% trifluoroacetic acid containing 10% of acetonitrile and 0.1% trifluoroacetic acid containing 50% of acetonitrile and the state of elution was monitored at the UV wavelength of 210 nm. As shown by the typical chromatogram in Fig. 7, the peak corresponding to HPCT can be clearly detected. The fractions containing such peaks were pooled and HPLC under the same conditions was performed again (Fig. 8) to obtain 2.15 mg (0.56 µ mole) of pure HPTC.

Structural identification of HPCT

The amino acid composition of HPCT was determined on the basis of the amino acid analysis of the hydrolysate of HPCT prepared by hydrolyzing HPCT with 6 N hydrochloric acid containing 0.1% of phenol in a sealed tube and the result of an amino acid analysis conducted after HPCT was oxidized with performic acid and hydrolyzed with 6 N HCl at 110°C for 24 hours in the conventional manner (1) (Table 1). The amino acid sequence of the performic acid oxidation product of HPCT was analyzed by Edman's degradation method (2), whereby the sequence to the 15th residue from the N-terminal could be determined (Fig. 9). Then, in order to determine the amino acid sequence of total HPCT, HPCT was degraded with trypsin in the hope of obtaining fragment peptides.

In 30 µl of 0.1 N PA buffer (pH 7.80) was suspended 160 µg of HPCT and after 30 minutes of ultrasonic treatment, 10 µl of TPCK-treated trypsin solution (3) (1 mg/ml) was added. The mixture was allowed to stand at 37°C for 2 hours. The reaction mixture was then lyophilized, dissolved in 400 µl of 0.1 N acetic acid, and subjected to HPLC. The conditions of HPLC used here were exactly identical with those used in the purification of HPCT and the chromatogram monitored at the UV wavelength of 210 nm was as reproduced in Fig. 10. Thus, the amino acid compositions of the peaks $T_1$, $T_2$ and $T_3$ suggested that $T_1$ was a peptide from the 19th amino acid unit of HPCT through the 34th amino acid unit, $T_2$ was a peptide from the N-terminal through the 18th amino acid unit, and $T_3$ was a peptide from the N-terminal through the 34th amino acid unit (Table 1).

The literature references mentioned in the foregoing are as follows.

(1) C. H. W. Hirs, "Methods in Enzymology", Academic Press, New York, Vol. 11, P. 197 (1967)

(2) J. P. Van Eerd et al., Biochemistry, *15*, 1171 (1976)

(3) S.-S. Wang et al., J. Biol. Chem., *240*, 1619 (1965)

Then, the amino acid sequence analysis was carried out using $T_1$ and the performic acid oxidation product $T_2$ (Fig. 9). Moreover, the amino acid sequence analysis of the C-terminal ends of HPCT, $T_1$, and $T_2$ was carried out by the carboxypeptidase method [T. Isobe et al.: J. Mol. Biol., *102*, 349 (1976)], and the C-terminal analysis thereof was carried out by the hydrazine method [A. Tsugita et al.: Proc. Nat. Acad. Sci., U.S.A., *46*, 1462 (1960)] (Fig. 9). Based on results of the above analyses, the amino acid sequence of HPCT was identified as shown in Fig. 9.

Purification of HPCT-Cp(1-33), the carboxypeptidase degradation product

In 300 µl of 0.1 N PA buffer (pH 7.80) was suspended 500 µg of HPCT and after 1 hour of ultrasonication, 7.5 µl of carboxypeptidase B solution (0.67 mg/ml) was added. The mixture was allowed to stand at 37°C for 6 hours. The liberated amino acids (2 moles of lysine and 1 mole of arginine) were detected by amino acid analysis. The reaction mixture was then lyophilized, dissolved in 500 µl of 0.1 acetic acid and purified by HPLC. The conditions of HPLC were identical with those used in the purification of HPTC and the chromatogram was monitored at the UV wavelength of 210 nm. Amino acid analysis of the peptide thus purified showed that it is a peptide from the N-terminal through the 33rd amino acid, i.e. HPCT·Cp(1-33) (Table 1). The yield calculated from the amino acid analysis data was 302 µg or 68%.

## Table 1

## The Amino Acid Compositions of HPCT, $T_1$, $T_2$ and HPCT-Cp(1-33)

|  | HPCT | $T_1$ | $T_2$ | HPCT-Cp(1-33) |
|---|---|---|---|---|
| CySO$_3$H | 1.6 |  | 1.8 | 1.9 |
| Asp | 3.1 |  | 3.0 | 3.0 |
| Thr | 4.7 | 1.8 | 2.8 | 4.7 |
| Ser | 1.1 |  | 1.1 | 1.0 |
| Glu | 2.0 | 1.0 | 1.1 | 2.1 |
| Pro | 2.0 | 1.9 |  | 1.9 |
| Gly | 4.8 | 2.8 | 2.0 | 5 |
| Ala | 2.2 | 1.9 |  | 2.1 |
| Val | 1.9 | 1.0 | 1.0 | 2.0 |
| Ile | 1.1 | 1.0 |  | 1.1 |
| Leu | 2 |  | 1.9 | 2.0 |
| Tyr | 1.0 |  | 1.0 | 1.0 |
| Phe | 2.7 | 1.9 | 1.1 | 3.0 |
| Lys | 2.9 | *1* | *1* | 1.1 |
| His | 0.9 | 0.9 |  | 1.0 |
| Arg | 1.0 |  |  |  |
|  | 36 | 16 | 18 | 33 |

In the table, the underscored figures were used to calculate the figures for other amino acids.

Biological activity of HPCT

The biological activities of HPCT and HPCT. Cp(1-33) purified by the above-mentioned procedure were assayed using the serum calcium lowering effect in rats as an indicator.

Procedure

Male Wistar strain rats weighing 300 to 350 grams were fasted overnight and used. Each animal was secured in supine position and under ether anesthesia, a polyethylene tube (Intramedic® PE-50, Clay Adams) filled with heparin sodium injection J. P. (Japan-Upjohn) was inserted into the femoral artery of the right hind leg. The rat was fixed in a Boleman cage, and 30 minutes after awakening of the animal, 0.5 ml of the blood was withdrawn through the above polyethylene tube inserted into the femoral artery into a polyethylene centrifugal tube containing 50 units of heparin sodium. Immediately thereafter 0.2 ml of the under-mentioned physiological saline, HPCT, HPCT·Cp(1-33) or swine calcitonin solution was administered subcutaneously at the back of the animal. Then, 1, 2, 4 and 6 hours after the administration, 0.5 ml each of the blood was taken through the above-mentioned polyethylene tube. The blood thus taken was promptly centrifuged on an Eppendorf microcentrifuge (Eppendorf 5414) at 13,000 r.p.m. for 1 minute and the supernatant was used as serum fraction.

The blood calcium level was measured by the o-cresolphthalein complexon method using a commercial calcium assay kit RM117-K (Ca SET) (Yatron). Fifty μl of the serum was taken in a test tube and stirred with 0.5 ml of the color reagent containing o-cresolphthalein complexon and 8-hydroxyquinoline (RM117-2). Then, 5.0 ml of monoethanolamine borate buffer (RM117-1) was added and stirred. Within 90 minutes thereafter, the absorbance was measured with a self-recording spectrophotometer (Hitachi 320) at the wavelength of 575 mμ. The concentration of calcium was calculated from this absorbance value.

The methods used in the preparation of the physiological saline solution, HPCT solution, HPCT·Cp(1-33) solution and swine calcitonin solution employed are as follows.

a) Physiological saline solution: Bovine serum albumin (Sigma®) was dissolved in physiolgical saline J. P. (Ohtsuka Pharmaceutical Co.) at room temperature to the albumin concentration of 0.1%.

b) HPCT solution: HPCT was diluted with the above-mentioned physiological saline solution containing 0.1% of albumin to the HPCT concentration of 4.5 μg/0.2 ml.

c) HPCT Cp(1-33) solution: HPCT Cp(1-33) was diluted with the above-mentioned physiological saline solution containing 0.1% of albumine to the HPCT Cp(1-33) concentration of 20 μg/0.2 ml.

d) Swine calcitonin solution: Calcital® for Injection (Yamanouchi Pharmaceutical Co.) containing 160 I.U. of calcitonin per vial was dissolved in 40 ml of a vehicle containing 16% of purified gelatin and 0.5% phenol J. P. and the solution was diluted with the above-mentioned physiological saline solution containing 0.1% of albumin. This dilution contained 0.2 or 1 I.U. of swine calcitonin per 0.2 ml.

Results

The results are shown in Table 2. The serum calcium level of the rate treated subcutaneously with 4.5 μg of HPCT dropped with statistical significance in 1 and 2 hours after the administration. The time course of serum calcium concentration after HPCT treatment was very similar to the time course of serum calcium observed in the rat subcutaneously given 0.2 I.U. of swine calcitonin. It was therefore considered that under the above experimental conditions 4.5 μg of HPCT is equivalent in activity to 0.2 I.U. of swine calcitonin.

The serum calcium level of the rat treated subcutaneously with 20 μg of HPCT·Cp(1-33) dropped with statistical significance in 1 hour after the administration, however, no dropping of the serum calcium level was observed since 2 hours after the administration.

With respect to calcitonins, it is known that compared with naturally-occurring peptides having amidated C-terminals, peptides with free C-terminals (desamidopeptides) are generally very low in biological activity. However, HPCT (a precursor of Val[8] human calcitonin) and HPCT Cp(1-33) suggest their stronger physiological activity as compared with swine calcitonin as shown in Table 2, though they can not be compared in an equal amount because the weight of the swine calcitonin in the Table is not clear.

Moreover, although much research has been done into the substitution of amino acids in the amino acid sequence of calcitonin and the partial elimination of peptides or amino acids from the sequence, it has never been known that the addition of a peptide as in this invention has ever given rise to an active substance.

## Table 2
### Infuluence of HPCT on Rat Serum Calcium

| | Change in serum Ca level (mg/dl) | | | |
|---|---|---|---|---|
| | 1 hour | 2 hours | 3 hours | 4 hours |
| Physiological saline | -0.01 ±0.03 | -0.12 ±0.08 | -0.31 ±0.08 | -0.45 ±0.07 |
| HPCT (4.5 µg/rat) | -0.85 b) ±0.09 | -0.64 b) ±0.08 | -0.47 ±0.05 | -0.63 ±0.11 |
| HPCT·Cp (1-33) (20 µg/rat) | -0.82 a) ±0.20 | -0.28 ±0.17 | -0.21 ±0.08 | -0.25 ±0.11 |
| Swine calcitonin (0.2 I.U./rat) | -0.70 a) ±0.19 | -0.71 b) ±0.07 | -0.49 ±0.06 | -0.60 ±0.12 |
| Swine calcitonin (1.0 I.U./rat) | -0.67 b) ±0.08 | -0.93 b) ±0.06 | -1.25 b) ±0.06 | -0.88 ±0.14 |

The figures are the mean values for 4 rats per group ± standard deviations.

a) Significant difference at $\leq 5\%$ level from physiological saline control

b) Significant difference at $\leq 1\%$ level from physiological saline control

EP 0 095 351 B1

**Claims**

1. A gene comprising a chemically synthesized polynucleotide which comprises the following sequence:

5'

```
TGT GGT AAC CTG AGC ACC TGT GTG CTG GGT ACC TAC ACC CAG

ACA CCA TTG GAC TCG TGG ACA CAC GAC CCA TGG ATG TGG GTC


GAT TTC AAC AAG TTC CAC ACC TTC CCG CAG ACC GCT ATC GGT

CTA AAG TTG TTC AAG GTG TGG AAG GGC GTC TGG CGA TAG CCA
```
3'
```
GTT GGT GCC CCG GGT AAG AAA CGC

CAA CCA CGG GGC CCA TTC TTT GCG,
```

the said sequence coding for the amino acid sequence represented by the formula:

```
Cys-Gly-Asn-Leu-Ser-Thr-Cys-Val-Leu-Gly-Thr-Tyr-

Thr-Gln-Asp-Phe-Asn-Lys-Phe-His-Thr-Phe-Pro-Gln-

Thr-Ala-Ile-Gly-Val-Gly-Ala-Pro-Gly-Lys-Lys-Arg.
```

2. A gene as claimed in claim 1, wherein a codon for methionine is added to the head of the said nucleotide sequence of the gene and translation-termination codons are added to the tail end.

3. A gene as claimed in claim 1 or claim 2, wherein one end of the gene is an EcoRI cohesive end and the other end is a BamHI cohesive end.

4. A gene as claimed in claim 1, wherein at least one of Rsal, Kpnl, Smal, and Xmal cleavage sites is provided in the nucleotide sequence.

5. A plasmid containing an *Escherichia coli* alkaline phosphatase of beta-galactosidase gene, which comprises a promoter region and a structural gene of the gene, one end of the gene having an EcoRI cleavage site and the other end having a BamHI cleavage site, and a gene comprising the following sequence:

5'

```
TGT GGT AAC CTG AGC ACC TGT GTG CTG GGT ACC TAC ACC CAG

ACA CCA TTG GAC TCG TGG ACA CAC GAC CCA TGG ATG TGG GTC


GAT TTC AAC AAG TTC CAC ACC TTC CCG CAG ACC GCT ATC GGT

CTA AAG TTG TTC AAG GTG TGG AAG GGC GTC TGG CGA TAG CCA
```
3'
```
GTT GGT GCC CCG GGT AAG AAA CGC

CAA CCA CGG GGC CCA TTC TTT GCG,
```

the said sequence coding for the following amino acid sequence;

Cys-Gly-Asn-Leu-Ser-Thr-Cys-Val-Leu-Gly-Thr-Tyr-

Thr-Gln-Asp-Phe-Asn-Lys-Phe-His-Thr-Phe-Pro-Gln-

Thr-Ala-Ile-Gly-Val-Gly-Ala-Pro-Gly-Lys-Lys-Arg.

6. A plasmid as claimed in claim 5, wherein the gene coding for the amino acid sequence is ligated to an *Escherichia coli* alkaline phosphatase or beta-galactosidase gene so that correct reading frame placement can be obtained.

7. An *Escherichia coli* transformant with a plasmid claimed in claim 5.

8. An *Escherichia coli* transformed strain as claimed in claim 7, which is E15/pAHPCT38 (FERM BP-283, Fermentation Research Institute).

9. A method of producing a precursor of calcitonin, which comprises (a) cultivating *Escherichia coli* cells transformed with a plasmid comprising an *Escherichia coli* alkaline phosphatase or beta-galactosidase structural gene attached to a synthetic gene comprising, in sequence, a codon for methionine, a polynucleotide represented by the sequence of

5'

TGT GGT AAC CTG AGC ACC TGT GTG CTG GGT ACC TAC ACC CAG

ACA CCA TTG GAC TCG TGG ACA CAC GAC CCA TGG ATG TGG GTC

GAT TTC AAC AAG TTC CAC ACC TTC CCG CAG ACC GCT ATC GGT

CTA AAG TTG TTC AAG GTG TGG AAG GGC GTC TGG CGA TAG CCA

3'

GTT GGT GCC CCG GGT AAG AAA CGC

CAA CCA CGG GGC CCA TTC TTT GCG

which encodes for the sequence of the amino acid precursor represented by the formula:

Cys-Gly-Asn-Leu-Ser-Thr-Cys-Val-Leu-Gly-Thr-Tyr-

Thr-Gln-Asp-Phe-Asn-Lys-Phe-His-Thr-Phe-Pro-Gln-

Thr-Ala-Ile-Gly-Val-Gly-Ala-Pro-Gly-Lys-Lys-Arg,

and a sequence which encodes for termination of translation, the transformant cells expressing the gene as a peptide comprising the calcitonin precursor and (b) recovering said calcitonin precursor from the cultured cells.

10. A method as claimed in claim 9, wherein one end of the synthetic gene is an EcoRI cleavage site and the other end is a BamHI cleavage site.

# EP 0 095 351 B1

**Patentansprüche**

1. Ein Gen, enthaltend ein chemisch-synthetisiertes Polynukleotid, welches die folgende Sequenz enthält:

5'

TGT GGT AAC CTG AGC ACC TGT GTG CTG GGT ACC TAC ACC CAG

ACA CCA TTG GAC TCG TGG ACA CAC GAC CCA TGG ATG TGG GTC

GAT TTC AAC AAG TTC CAC ACC TTC CCG CAG ACC GCT ATC GGT

CTA AAG TTG TTC AAG GTG TGG AAG GGC GTC TGG CGA TAG CCA

3'

GTT GGT GCC CCG GGT AAG AAA CGC

CAA CCA CGG GGC CCA TTC TTT GCG,

wobei die genannte Sequenz für die in der nachfolgenden Formel wiedergegebene Aminosäuresequenz kodiert:

Cys-Gly-Asn-Leu-Ser-Thr-Cys-Val-Leu-Gly-Thr-Tyr-

Thr-Gln-Asp-Phe-Asn-Lys-Phe-His-Thr-Phe-Pro-Gln-

Thr-Ala-Ile-Gly-Val-Gly-Ala-Pro-Gly-Lys-Lys-Arg.

2. Ein Gen gemäß Anspruch 1, dadurch gekennzeichnet, daß ein Kodon für Methionin am Kopfende der Nukleotidsequenz des Gens hinzugefügt ist und Translations-Terminationskodons am Schwanzende hinzugefügt sind.

3. Ein Gen gemäß den Ansprüchen 1 oder 2, dadurch gekennzeichnet, daß ein Ende des Gens ein EcoRI kohäsives Ende und das andere Ende ein BamHI kohäsives Ende ist.

4. Ein Gen gemäß Anspruch 1, dadurch gekennzeichnet, daß mindestens eine der RsaI-, KpnI-, SmaI- und XmaI-Schnittstellen in der Nukleotidsequenz vorhanden ist.

5. Ein Plasmid, das ein *Escherichia coli* alkalische Phosphatase- oder beta-Galactosidase-Gen enthält, welches einen Promotorbereich und ein Strukturgen des Gens umfaßt, wobei ein Ende des Gens eine EcorRI-Schnittstelle und das andere Ende eine BamHI-Schnittstelle aufweisen und das Gen die folgende Sequenz enthält:

5'

TGT GGT AAC CTG AGC ACC TGT GTG CTG GGT ACC TAC ACC CAG

ACA CCA TTG GAC TCG TGG ACA CAC GAC CCA TGG ATG TGG GTC

GAT TTC AAC AAG TTC CAC ACC TTC CCG CAG ACC GCT ATC GGT

CTA AAG TTG TTC AAG GTG TGG AAG GGC GTC TGG CGA TAG CCA

3'

GTT GGT GCC CCG GGT AAG AAA CGC

CAA CCA CGG GGC CCA TTC TTT GCG,

15

wobei diese Sequenz für die folgende Aminosäuresequenz kodiert:

```
Cys-Gly-Asn-Leu-Ser-Thr-Cys-Val-Leu-Gly-Thr-Tyr-

Thr-Gln-Asp-Phe-Asn-Lys-Phe-His-Thr-Phe-Pro-Gln-

Thr-Ala-Ile-Gly-Val-Gly-Ala-Pro-Gly-Lys-Lys-Arg.
```

6. Plasmid gemäß Anspruch 5, dadurch gekennzeichnet, daß das für die Aminosäuresequenz kodierende Gen mit einem *Escherichia coli* alkalische Phosphatase- oder beta-Galactosidase-Gen auf solche Weise verknüpft ist, daß die richtige Plazierung des Leserahmens erhalten werden kann.

7. Ein *Escherichia coli* Transformant mit einem Plasmid gemäß Anspruch 5.

8. Ein transformierter *Escherichia coli* Stamm gemäß Anspruch 7, bei dem es sich um E15/pAHPCT38 (FERM BP-283, Fermentation Research Institute) handelt.

9. Verfahren zur Herstellung einer Vorstufe von Calcitonin, dadurch gekennzeichnet, daß man (a) *Escherichia coli* Zellen kultiviert, die mit einem Plasmid transformiert sind, welches ein *Escherichia coli* alkalische Phosphatase oder beta-Galactosidase Struktur-Gen enthält, welches mit einem synthetischen Gen verknüpft ist, welches in Sequenz ein Kodon für Methionin, ein Polynukleotid der Sequenz

```
5'

TGT GGT AAC CTG AGC ACC TGT GTG CTG GGT ACC TAC ACC CAG

ACA CCA TTG GAC TCG TGG ACA CAC GAC CCA TGG ATG TGG GTC


GAT TTC AAC AAG TTC CAC ACC TTC CCG CAG ACC GCT ATC GGT

CTA AAG TTG TTC AAG GTG TGG AAG GGC GTC TGG CGA TAG CCA

                                    3'

GTT GGT GCC CCG GGT AAG AAA CGC

CAA CCA CGG GGC CCA TTC TTT GCG
```

welches für die Sequenz der Aminosäurenvorstufe der Formel

```
Cys-Gly-Asn-Leu-Ser-Thr-Cys-Val-Leu-Gly-Thr-Tyr-

Thr-Gln-Asp-Phe-Asn-Lys-Phe-His-Thr-Phe-Pro-Gln-

Thr-Ala-Ile-Gly-Val-Gly-Ala-Pro-Gly-Lys-Lys-Arg
```

kodiert,
und eine Sequenz aufweist, die für die Termination der Translation kodiert, wobei die Transformantenzellen das Gen als ein Peptid exprimieren, welches die Calcitonin-Vorstufe enthält und (b) die Calcitonin-Vorstufe aus den kultivierten Zellen gewinnt.

10. Verfahren gemäß Anspruch 9, dadurch gekennzeichnet, daß das eine Ende des synthetischen Gens eine EcoRI-Schnittstelle und das andere Ende eine BamHI-Schnittstelle sind.

**Revendications**

1. Gène contenant un polynucléotide synthétisé par voie chimique qui contient la séquence suivante:

5'

TGT GGT AAC CTG AGC ACC TGT GTG CTG GGT ACC TAC ACC CAG

ACA CCA TTG GAC TCG TGG ACA CAC GAC CCA TGG ATG TGG GTC

GAT TTC AAC AAG TTC CAC ACC TTC CCG CAG ACC GCT ATC GGT

CTA AAG TTG TTC AAG GTG TGG AAG GGC GTC TGG CGA TAG CCA

3'

GTT GGT GCC CCG GGT AAG AAA CGC

CAA CCA CGG GGC CCA TTC TTT GCG,

ladite séquence codant pour la séquence d'amino-acides représentée par la formule:

Cys-Gly-Asn-Leu-Ser-Thr-Cys-Val-Leu-Gly-Thr-Tyr-

Thr-Gln-Asp-Phe-Asn-Lys-Phe-His-Thr-Phe-Pro-Gln-

Thr-Ala-Ile-Gly-Val-Gly-Ala-Pro-Gly-Lys-Lys-Arg.

2. Gène selon la revendication 1, dans lequel un codon pour la méthionine est ajouté en tête de ladite séquence de nucléotides du gène et des codons d'arrêt de traduction sont ajoutés en queue.

3. Gène selon la revendication 1 ou 2, dans lequel une extrémité du gène est une extrémité cohésive EcoRI et l'autre extrémité est une extrémité cohésive BamHI.

4. Gène selon la revendication 1, dans lequel au moins un des sites de coupure RsaI, KpnI, SmaI et XmaI est prévu dans la séquence de nuclétodides.

5. Plasmide contenant une gène de phosphates alcaline ou de ß-galactosidase d'*Escherichia coli,* qui comprend une région de promoteur et un gène de structure du gène, une extrémité du gène ayant un site de coupure EcoRI et l'autre extrémité ayant un site de coupure BamHI, et un gène comprenant la séquence suivante:

5'

TGT GGT AAC CTG AGC ACC TGT GTG CTG GGT ACC TAC ACC CAG

ACA CCA TTG GAC TCG TGG ACA CAC GAC CCA TGG ATG TGG GTC

GAT TTC AAC AAG TTC CAC ACC TTC CCG CAG ACC GCT ATC GGT

CTA AAG TTG TTC AAG GTG TGG AAG GGC GTC TGG CGA TAG CCA

3'

GTT GGT GCC CCG GGT AAG AAA CGC

CAA CCA CGG GGC CCA TTC TTT GCG,

**EP 0 095 351 B1**

ladite séquence codant pour la séquence d'amino-acides suivante:

        Cys-Gly-Asn-Leu-Ser-Thr-Cys-Val-Leu-Gly-Thr-Tyr-

        Thr-Gln-Asp-Phe-Asn-Lys-Phe-His-Thr-Phe-Pro-Gln-

        Thr-Ala-Ile-Gly-Val-Gly-Ala-Pro-Gly-Lys-Lys-Arg.

6. Plasmide selon la revendication 1, dans lequel le gène codant pour la séquence d'amino-acides est lié à un gène de phosphatase alcaline ou de ß-galactosidase d'*Escherichia coli* de façon à pouvoir obtenir un placement correct du cadre de lecture.

7. Transformant d'*Escherichia coli* avec un plasmide selon la revendication 5.

8. Souche transformée d'*Escherichia coli* selon la revendication 7, qui est E15/pAHPCT38 (FERM BP-283, Fermentation Research Institute).

9. Procédé de production d'un précurseur de calcitonine, qui consiste (a) à cultiver des cellules d'*Escherichia coli* transformées avec un plasmide comprenant un gène de structure de phosphatase alcaline ou de ß-galactosidase d'*Escherichia coli* fixé à un gène synthétique comprenant, en séquence, un codon pour la méthionine, un polynucléotide représenté par la séquence de

    5'

    TGT GGT AAC CTG AGC ACC TGT GTG CTG GGT ACC TAC ACC CAG

    ACA CCA TTG GAC TCG TGG ACA CAC GAC CCA TGG ATG TGG GTC


    GAT TTC AAC AAG TTC CAC ACC TTC CCG CAG ACC GCT ATC GGT

    CTA AAG TTG TTC AAG GTG TGG AAG GGC GTC TGG CGA TAG CCA

                                        3'

    GTT GGT GCC CCG GGT AAG AAA CGC

    CAA CCA CGG GGC CCA TTC TTT GCG

qui code pour la séquence du précurseur d'amino-acides représentée par la formule

        Cys-Gly-Asn-Leu-Ser-Thr-Cys-Val-Leu-Gly-Thr-Tyr-

        Thr-Gln-Asp-Phe-Asn-Lys-Phe-His-Thr-Phe-Pro-Gln-

        Thr-Ala-Ile-Gly-Val-Gly-Ala-Pro-Gly-Lys-Lys-Arg,

et une séquence qui code pour l'arrêt de la traduction, les cellules transformantes exprimant le gène sous forme d'un peptide comprenant le précurseur de calcitonine, et (b) à recueillir ledit précurseur de calcitonine à partir des cellules cultivées.

10. Procédé selon la revendication 9, dans lequel une extrémité du gène synthétique est un site de coupure EcoRI et l'autre extrémité est un site de coupure BamHI.

# Fig. 1

H-Cys-Gly-Asn-Leu-Ser-Thr-Cys-Val-Leu-Gly-Thr-Tyr-Thr-Gln-Asp-Phe-Asn-Lys-    8  10  15

Phe-His-Thr-Phe-Pro-Gln-Thr-Ala-Ile-Gly-Val-Gly-Ala-Pro-Gly-Lys-Lys-Arg-OH    20  25  30  32  36

8 — Val ——————————— Pro-NH2    32

8 — Met ——————————— Pro-NH2    32

EP 0 095 351 B1

# Fig. 2

```
        1               5                    10                   15
Met-Cys-Gly-Asn-Leu-Ser-Thr-Cys-Val-Leu-Gly-Thr-Tyr-Thr-Gln-Asp-Phe-Asn-Lys-Phe-
        1               3               5                    7                9
AATTC ATG TGT GGT AAC CTG AGC ACC TGT GTG CTG GGT ACC TAC ACC CAG GAT TTC AAC AAG TTC
    G TAC ACA CCA TTG GAC TCG TGG ACA CAC GAC CCA TGG ATG TGG GTC CTA AAG TTG TTC AAG
                2               4                    6                    8           10
```

```
     20                     25                   30     32              36
His-Thr-Phe-Pro-Gln-Thr-Ala-Ile-Gly-Val-Gly-Ala-Pro-Gly-Lys-Lys-Arg-stop-stop
         11            13              15             17              19
CAC ACC TTC CCG CAG ACCGCT ATC GGT GTT GGT GCC CCG GGT AAG AAA CGC TAA TAG
GTG TGG AAG GGC GTC TGG CGA TAG CCA CAA CCA CGG GGC CCA TTC TTT GCG ATT ATC CTAG
        12            14               16             18              20
```

## Fig. 3

| A | B | C |
|---|---|---|
| F 1) AATTCATGTGT | F 8) GAAATCCTGGGT | F 15) ATCGGTGTTGGT |
| F 2) GTTACCACACATG | F 9) GATTTCAACAAG | F 16) CGGGGCACCAAC |
| F 3) GGTAACCTGAGC | F 10) GTGGAACTTGTT | F 17) GCCCCGGGTAAG |
| F 4) ACAGGTGCTCAG | F 11) TTCCACACCTTC | F 18) CGTTTCTTACC |
| F 5) ACCTGTGTGCTG | F 12) CTGCGGGAAGGT | F 19) AAACGCTAATAG |
| F 6) GTAGGTACCCAGCAC | F 13) CCGCAGACCGCT | F 20) GATCCTATTAG |
| F 7) GGTACCTACACCCAG | F 14) ACCGATAGCGGT | |

EP 0 095 351 B1

Fig. 4

# Fig. 5

n = 0 or 1

# Fig. 6

# Fig. 7

# Fig. 8

# Fig. 9

Cys-Gly-Asn-Leu-Ser-Thr-Cys-Val-Leu-Gly-Thr-Tyr-Thr-Gln-Asp-Phe-Asn-Lys-

HPCT

T2

CpAB
CpP

Phe-His-Thr-Phe-Pro-Gln-Thr-Ala-Ile-Gly-Val-Gly-Ala-Pro-Gly-Lys-Lys-Arg

HPCT

CpB

T1

CpB

# Fig. 10

Fig. 11

200 →

150 →
140 →

118 →

100 →

82 →

66 →

# Fig. 12